# EUROPEAN PATENT APPLICATION

(11) **EP 4 506 026 A1**
(43) Date of publication of application: **12.02.2025**
(21) Application number: 23796712.0
(22) Date of filing: 21.04.2023
(51) Int. Cl.: A61M 5/168, A61M 5/142

(54) **DRUG INJECTOR MONITORING METHOD AND DEVICE**

(30) Priority: 28.04.2022 KR 20220052512
(71) Applicant: Caremedi Co., Ltd., Seoul 07207 (KR); Sogang University Research & Business Development Foundation, Seoul 04107 (KR)
(72) Inventor: SHIN, Woonsup, Seoul 04065 (KR); ZHU, Enhua, Seoul 04109 (KR); SHIN, Yeong Jong, Gimpo-si Gyeonggi-do 10126 (KR); LEE, Junghyun, Seoul 03916 (KR)
(74) Representative: Manitz Finsterwald Patent- und Rechtsanwaltspartnerschaft mbB
(86) International application number: PCT/KR2023/005451
(87) International publication number: WO 2023/211065

(57) **Abstract**

One embodiment of the present disclosure relates to drug injector monitoring method and device and provides drug injector monitoring method and device that monitor a battery state, a remaining drug amount, temperature, and pressure of a drug injector such that a fixed amount of drug may be stably injected.

## Description

### BACKGROUND

The present disclosure relates to drug injector monitoring method and device, and more specifically, to a method and device for monitoring a safety state of a drug injector.

Depending on types, purposes of treatment, methods, and so on, drug may be injected into the body in various forms, such as oral, subcutaneous, and intravenous administration. A drug injector using a drug pump may automatically inject drug into the body at a desired speed and dose at a required time. Therefore, a drug injector using a drug pump may be used not only in hospitals or in a patient's daily living environment, but also in various forms.

An insulin pump, which is commonly called an insulin injector, is for diabetics who do not secrete insulin or secrete only small amounts of insulin and is a medical device that supplies insulin into the body accurately at a set time from the outside to adjust blood sugar.

The insulin pump is a technology used for insulin-dependent diabetes patients. Therefore, an insulin pump may be attached to a patient to continuously inject drugs into the patient for 24 hours. As drug is continuously injected into a patient, when the exact amount of drugs is not stably injected, the patient's health may be adversely affected.

The reasons why drug is not stably injected through a pump is due to various reasons, such as malfunction of the pump and blockage of a drug injection hole. Therefore, it is necessary to develop technology that may comprehensively monitor injection of drug.

### SUMMARY

The present disclosure provides method and device for monitoring a safety state of a drug injector.

Technical objects to be achieved by the present disclosure are not limited to the technical objects described above, and there may be other technical objects.

According to an aspect of the present disclosure, a drug injector monitoring method performed by a processor includes obtaining at least one or more pieces of state information on an operation of a drug injector from the drug injector, and outputting one of a notification signal on a state of the drug injector and a control signal for stopping a drug injection operation of the drug injector based on a result of comparing the state information with a reference value for each piece of the state information, wherein the state information includes a battery remaining amount of the drug injector, a drug remaining amount of the drug injector, temperature of a specific region in the drug injector, and or pressure of the specific region in the drug injector.

According to another aspect of the present disclosure, a drug injector monitoring device includes a memory storing a drug injector monitoring program, and a processor configured to execute the drug injector monitoring program stored in the memory, wherein the processor is further configured to execute the drug injector monitoring program to obtain at least one or more pieces of state information on an operation of a drug injector from the drug injector and to output one of a notification signal on a state of the drug injector and a control signal for stopping a drug injection operation of the drug injector based on a result of comparing the state information with a reference value for each piece of the state information, and the state information includes a battery remaining amount of the drug injector, a drug remaining amount of the drug injector, temperature of a specific region in the drug injector, and or pressure of the specific region in the drug injector.

Drug injector monitoring method and device according to embodiments of the present disclosure may detect whether a drug injector is in a state of being able to stably inject drug by sensing pressure, temperature, a battery remaining amount, and a drug remaining amount of the drug injector, and thus, it is possible to prevent an inaccurate amount of drug from being injected into a user and to determine whether the drug injector is abnormal.

### BRIEF DESCRIPTION OF THE DRAWINGS

Embodiments of the inventive concept will be more clearly understood from the following detailed description taken in conjunction with the accompanying drawings in which:
FIG. 1 is a schematic diagram of a drug injection monitoring system according to an embodiment of the present disclosure;
FIG. 2 is a configuration diagram of a user terminal and a drug injector according to an embodiment of the present disclosure;
FIG. 3 is a configuration diagram of a processor according to an embodiment of the present disclosure;
FIG. 4 is a configuration diagram of a processor according to another embodiment of the present disclosure;
FIG. 5 is an example diagram of a drug injector according to an embodiment of the present disclosure;
FIG. 6 is an example diagram of an electroosmotic pump according to an embodiment of the present disclosure;
FIG. 7 is a flowchart of a drug injector monitoring method according to an embodiment of the present disclosure;
FIG. 8 is a flowchart of a drug injection operation stop and alarm notification generation method according to an embodiment of the present disclosure;
FIG. 9 is a flowchart of a drug injection operation stop and alarm notification generation method according to another embodiment of the present disclosure;
FIG. 10 is a flowchart of a drug injection operation stop and alarm notification generation method according to another embodiment of the present disclosure; and
FIG. 11 is a flowchart of a drug injection operation stop and alarm notification generation method according to another embodiment of the present disclosure.

### DETAILED DESCRIPTION OF THE EMBODIMENTS

Hereafter, the present disclosure will be described in detail with reference to the accompanying drawings. However, the present disclosure may be implemented in many different forms and is not limited to the embodiments of the present disclosure described herein. In addition, the accompanying drawings are only for easy understanding of the embodiments of the present disclosure disclosed in the present specification, and the technical ideas disclosed in the present specification are not limited by the accompanying drawings. All terms which include technical and scientific terms used herein, should be interpreted as having a meaning generally understood by a person of ordinary skill in the art to which the present disclosure belongs. Terms defined in advance should be interpreted as having additional meanings consistent with the relevant technical literature and the presently disclosed content, and should not be interpreted in a very ideal or restrictive sense unless otherwise defined.

In order to clearly describe the present disclosure in the drawings, parts irrelevant to the descriptions are omitted, and a size, a shape, and a form of each component illustrated in the drawings may be variously modified. The same or similar reference numerals are assigned to the same or similar portions throughout the specification.

Suffixes "module" and "unit" for the components used in the following description are given or used interchangeably in consideration of ease of writing the specification, and do not have meanings or roles that are distinguished from each other by themselves. In addition, in describing the embodiments of the present disclosure disclosed in the present specification, when it is determined that a detailed descriptions of related known technologies may obscure the gist of the embodiments disclosed in the present specification, the detailed descriptions are omitted.

Throughout the specification, when a portion is said to be "connected (coupled, in contact with, or combined)" with another portion, this includes not only a case where it is "directly connected (coupled, in contact with, or combined)" ", but also a case where there is another member therebetween. In addition, when a portion "includes (comprises or provides)" a certain component, this does not exclude other components, and means to "include (comprise or provide)" other components unless otherwise described.

Terms indicating ordinal numbers, such as first and second, used in the present specification are used only for the purpose of distinguishing one component from another component and do not limit the order or relationship of the components. For example, the first component of the present disclosure may be referred to as the second component, and similarly, the second element may also be referred to as the first component. Singular forms used herein should be construed to include plural forms, unless the opposite is clearly indicated.

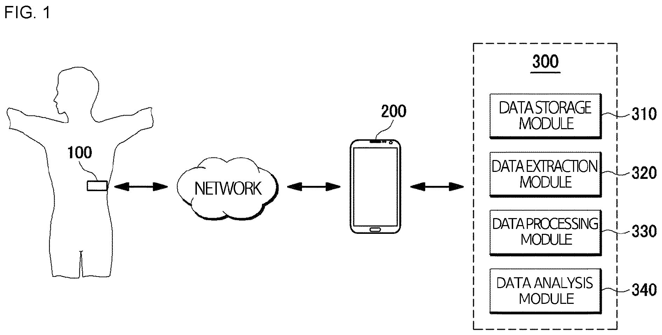

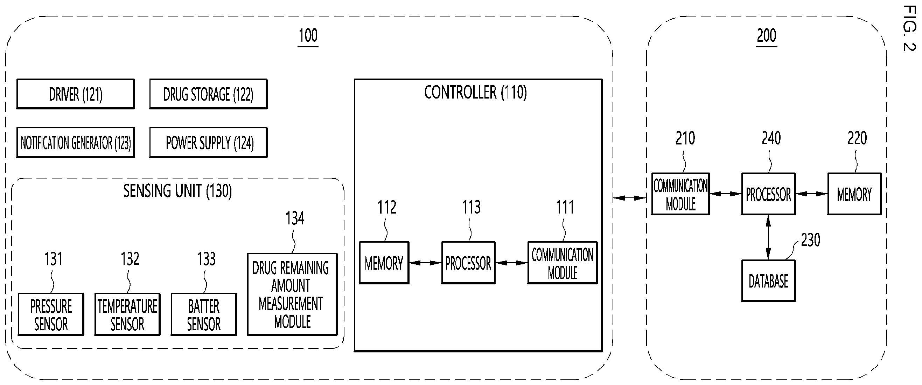

Hereinafter, a drug injection monitoring system (hereinafter, referred to as a "drug injector monitoring system") according to an embodiment of the present disclosure will be described with reference to FIGS. 1 and 2.

The drug injector monitoring system includes a drug injector 100 and a user terminal 200. The drug injector 100 refers to a device for injecting a drug to a patient or user. The drug injector 100 may include an insulin patch or an insulin pump that may be attached to a user to inject a drug into the user, as well as other devices for injecting a drug.

The user terminal 200 may refer to a terminal device for generating a control signal of the drug injector 100 or performing state monitoring of the drug injector 100. The user terminal 200 may include a display for displaying data to a user, and may display a notification to the user by using display, sound, vibration, a low-frequency stimulation signal, or so on. The drug injector 100 may inject a drug according to a control signal generated by the user terminal 200.

The user terminal 200 may mean any type of handheld-based wireless communication device, such as a desktop computer or a laptop computer equipped with a web browser, a wireless communication device that ensures portability and mobility, a smartphone, or a tablet personal computer (PC).

The drug injector 100 may generate a pulse sequence by using a drug injection control signal generated by the user terminal 200 and perform a drug injection operation according to the generated pulse sequence. In another embodiment, the user terminal 200 may generate a pulse sequence, and the drug injector 100 may perform a drug injection operation according to the pulse sequence generated by the user terminal 200.

The drug injector 100 provides data related to monitoring of the drug injector 100 to the user terminal 200 through a communication connection with the user terminal 200, and receives a control signal from the user terminal 200. A communication network illustrated in FIG. 1 may be implemented by a wired network, such as a local area network (LAN), a wide area network (WAN), or a value added network (VAN), or any type of wireless communication networks, such as a mobile radio communication network and a satellite communication network. In addition, the wireless communication networks may include, for example, third generation (3G), fourth generation (4G), fifth generation (5G), third generation partnership project (3GPP), long term evolution (LTE), world interoperability for microwave access (WIMAX), Wi-Fi, Bluetooth communication, infrared communication, ultrasonic communication, visible light communication (VLC), LiFi, and so on but are not limited thereto.

In another embodiment, the drug injector monitoring system may further include a data storage 300 in addition to the drug injector 100 and the user terminal 200. The data storage 300 may receive data from the user terminal 200 and accumulatively store data for a certain period of time. In addition, the data storage 300 may perform data analysis and processing functions, such as machine learning or user-customized injection algorithm generation, by using information on drug injection, such as a drug injection history, a drug injection result, and user information.

The data storage 300 may include a data storage module 310, a data extraction module 320, a data processing module 330, and a data analysis module 340 to perform the functions described above. The data storage module 310 performs a function of storing information on drug injection, such as user information, a drug injection history, and a drug injection result, from the drug injector 100 or the user terminal 200.

The data extraction module 320 may extract data related to drug injection from the data storage module 310, public data, big data, or so on, or perform a function of classifying the stored data according to set criteria. The data extraction module 320 may generate data statistics related to a user's drug injection pattern and drug injection and provide the data statistics to the user.

The data processing module 330 may perform preprocessing by using the data acquired from the data storage module 310 or the data extraction module 320, or may perform a data filtering function or a conversion function into data related to drug injection. The data analysis module 340 may perform machine learning to generate a user-customized injection algorithm by using the data acquired from the data storage module 310, the data extraction module 320, and the data processing module 330.

The data storage 300 may be configured in the form of a device, such as a server or a terminal, and may operate in a cloud computing service model, such as software as a service (SaaS), platform as a service (PaaS), or Infrastructure as a Service (laaS). In addition, the data storage 300 may be constructed in the form of a server, such as a private cloud system, a public cloud system, or a hybrid cloud system.

FIG. 2 is a configuration diagram of a drug injector and a user terminal according to an embodiment of the present disclosure.

Referring to FIG. 2, the drug injector 100 includes a controller 110, a driver 121, a drug storage 122, a notification generator 123, a power supply 124, and a sensing unit 130.

The controller 110 performs a function of controlling an operation of the drug injector 100. The controller 110 may include a communication module 111, a memory 112, and a processor 113.

The communication module 111 performs information transmission and reception with the user terminal 200. The communication module 111 may refer to a device including hardware and software required to transmit and receive signals, such as control signals or data signals, to and from other network devices through wired or wireless connections. The communication module 111 may receive data used for a drug injector control program from the user terminal 200, or transmit an operation control signal to the user terminal 200.

The memory 112 stores the drug injector control program and the drug injector monitoring program. The names of the drug injector control program and the drug injector monitoring program are set for the sake of convenience of description, and the names do not limit functions of the programs. The memory 112 may store at least one of information and data input to the communication module 111, information and data required for functions performed by the processor 113, and data generated according to execution of the processor 113.

The processor 113 is configured to execute the drug injector control program stored in the memory 112. The processor 113 may include various types of devices that control and process data. The processor 113 may refer to a data processing device which is built in hardware and includes a physically structured circuit to perform a function represented by a code or command included in a program. The processor 113 may be implemented by a microprocessor, a central processing unit (CPU), a processor core, a multiprocessor, an application-specific integrated circuit (ASIC), a field programmable gate array (FPGA), or so on, but the scope of the present disclosure is not limited thereto.

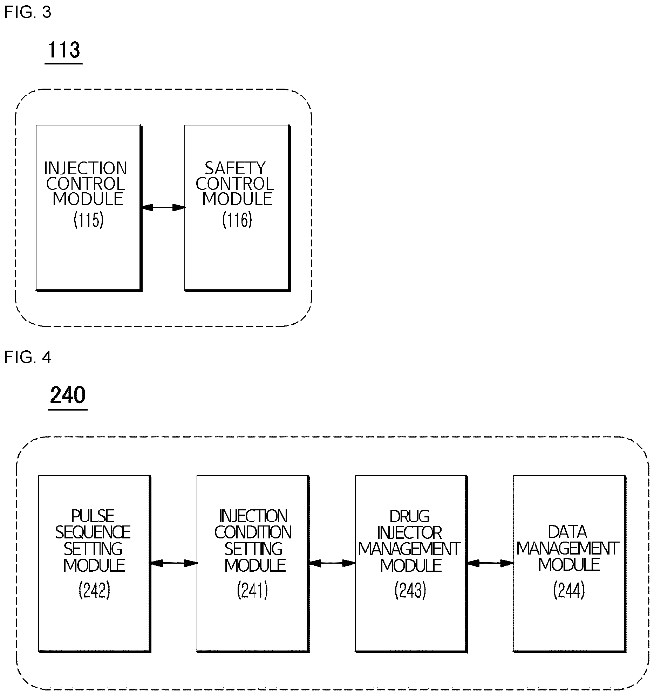

The processor 113 may control operations of the driver 121, the drug storage 122, the notification generator 123, the power supply 124, and the sensing unit 130. In addition, the processor 113 may generate a pulse sequence such that the drug injector 100 injects a fixed amount of drug, and may control a drug injection operation of the drug injector 100 according to a pulse sequence. A specific function of the processor 113 is described below in detail with reference to FIG. 3.

The driver 121 may include a drug injection pump, which is a pressure generator that generates pressure for performing drug injection according to a drug injection control signal, a needle and a cannula for injecting drug into a user's body, and so on. The configuration of the driver 121 is not limited thereto, and may refer to all configurations for performing drug injection according to a type of the drug injector 100.

The notification generator 123 may generate a notification to be provided to a user when an abnormality occurs in relation to the drug injector 100, such as when the drug injector 100 may not perform a drug injection operation, when there is a possibility that an error greater than or equal to a preset reference value may occur between the set drug injection amount and the actual drug injection amount, or when the drug injection has to be stopped immediately.

The notification generator 123 may use a method of generating a notification by using a method by which a user's attention may be drawn, such as sound, vibration, or low-frequency stimulation. In addition, the notification generator 123 may display to a user the types of notifications, such as an alarm notification generated when drug injection of the drug injector 100 has to be stopped immediately, a caution notification to notify the user in advance when the drug injector 100 approaches a condition for stopping the drug injection, and a notification display step for simply displaying a current state of the drug injector 100.

The sensing unit 130 measures data for monitoring the operating status of the drug injector 100 so that the drug injector 100 may inject an accurate amount of drug in a stable state. The sensing unit 130 may include a pressure sensor 131, a temperature sensor 132, a battery sensor 133, a drug remaining amount measurement module 134, and so on.

The pressure sensor 131 measures pressure of the drug injector 100. For example, the pressure sensor 131 may directly or indirectly measure the pressure of the driver 121 that generates pressure in the drug injector 100 or pressure of a path through which drug moves. The temperature sensor 132 measures temperature of the drug injector 100. For example, the temperature sensor 132 measures temperature of a pressure generator (the driver 122) that generates pressure in the drug injector 100.

The battery sensor 133 measures the remaining battery capacity of the drug injector 100. A function of the battery sensor 133 is not limited thereto, and may further include a function of deriving a remaining power capacity, a remaining operating time, or so on of the power supply 124.

The drug remaining amount measurement module 134 may directly or indirectly measure the drug remaining amount of the drug storage 122. For example, the drug remaining amount measurement module 134 may perform a function of calculating the drug remaining amount by using a drug storage amount initially stored in the drug storage 122 and a drug amount discharged by the drug injection operation. In addition, the drug remaining amount measurement module 134 may sense not only a remaining amount by using the volume of a remaining drug but also the remaining amount by using characteristics of drug, such as a weight of the remaining drug.

The user terminal 200 may display information on the drug injector 100 to a user. In addition, the user terminal 200 may receive injection-related data from a user and generate a control signal of the drug injector 100 or manage data related to drug injection. The user terminal 200 may be configured to include a communication module 210, a memory 220, and a processor 240, and may further include a database 230.

The communication module 210 transmits and receives information to and from the drug injector 100. The communication module 210 may refer to a device including hardware and software required to transmit and receive signals, such as control signals or data signals, to and from other network devices through wired or wireless connections. The communication module 210 may receive data used for a drug injector control program from the drug injector 100, or transmit a control signal related to an operation to the drug injector 100. In addition, the communication module 210 may receive data related to an operation of the drug injector 100, and the user terminal 200 may display the received data to a user through the display.

The memory 220 stores a drug injector control program and a drug injector monitoring program. The names of the drug injector control program and the drug injector monitoring program are set for the sake of convenience of description, and the names do not limit the functions of the programs. The memory 220 may store at least one of information and data input to the communication module 210, information and data required for functions performed by the processor 240, and data generated according to execution of the processor 240.

The memory 220 should be interpreted as a general term for a nonvolatile storage device that maintains the stored information even when power is not supplied and a volatile storage device that requires power to maintain the stored information. In addition, the memory 220 may perform a function of temporarily or permanently storing the data processed by the processor 240. The memory 220 may include magnetic storage media or flash storage media in addition to the volatile storage device that requires power to maintain the stored information, but the scope of the present disclosure is not limited thereto.

The database 230 may refer to a configuration in which operation data of the drug injector 100, data required for monitoring the drug injector 100, data for machine learning, big data, and so on are stored. The database 230 may constitute a part of the memory 220 but may be located the outside without being necessarily located inside the user terminal 200.

The processor 240 is configured to execute the drug injector control program stored in the memory 220. The processor 240 may include various types of devices that control and process data. The processor 240 may refer to a data processing device which is built in hardware and includes a physically structured circuit to perform a function represented by a code or command included in a program. The processor 240 may be implemented by a microprocessor, a CPU, a processor core, a multiprocessor, an ASIC, a FPGA, or so on, but the scope of the present disclosure is not limited thereto.

The processor 240 may execute a drug injector control program to generate a control signal for the drug injector 100, calculate a drug injection amount in response to a user's input, or manage data related to monitoring of a state of the drug injector 100 and drug injection. A specific function of the processor 240 is described below in detail with reference to FIG. 4.

FIG. 3 is a conceptual diagram illustrating a function of the processor 113 according to an embodiment of the present disclosure. An operation of the processor 113 according to the embodiment of the present disclosure is described below with reference to FIG. 3.

The processor 113 may include an injection control module 115 and a safety control module 116. The processor 113 may perform a function of generating a control signal to control the driver 121 by receiving a drug injection signal generated by using a drug injector control program stored in the memory 112. In addition, the processor 113 may monitor whether the drug injector 100 may operate stably, control an operation of the drug injector 100, or generate an alarm and notification.

The injection control module 115 may control the operation of the drug injector 100 by converting the drug injection signal generated by the user terminal 200 into a signal for controlling the driver 121. In this case, the injection control module 115 may perform or execute a function or program for controlling the operation of the drug injector 100. The injection control module 115 may perform not only a function of simply performing signal conversion, but also a function of generating a control signal of the driver 121 by receiving a drug injection amount for each injection mode or a drug injection speed (injection amount) per hour, an injection period, and so on from the user terminal 200 and by using the drug injection amount for each injection mode or the drug injection speed (injection amount) per hour, the injection period, and so on.

Here, the injection mode may include a base injection mode, a temporary base injection mode, and an immediate injection mode. The base injection mode may refer to an injection mode in which drug is continuously injected at a slow speed. That is, the base injection mode refers to an injection mode in which drug is injected at a base injection speed for a certain period of time to maintain a user's blood sugar level.

The temporary base injection mode refers to an injection mode in which an injection speed of the base injection mode is temporarily changed for a short period of time or a certain period of time. Therefore, the temporary base injection mode refers to an injection mode that performs drug injection in response to a temporary change in a user's blood sugar level, such as when a user plans to exercise or when the user's blood sugar level temporarily decreases due to the exercise.

The immediate injection mode refers to an injection method by which a bolus injection amount of drug is injected into the shortest time. Therefore, the immediate injection mode refers to an injection mode including a correction bolus for injecting drug into a user to lower the user's blood sugar level when the user's blood sugar level suddenly increases, and a meal bolus for injecting drug into the user before a meal or snack to maintain the user's blood sugar level that is increased or is expected to increase after the user eats within a target blood sugar level range.

In addition, the injection control module 115 may set priority for the base injection mode, the temporary base injection mode, and the immediate injection mode described above. The injection control module 115 may set priority between the injection modes, thereby being able to perform a drug injection operation according to the priority even when the drug injector 100 receives multiple drug injection signals having different injection modes.

The injection control module 115 may generate a pulse sequence for controlling the driver 121 according to the drug injection signal generated by the user terminal 200. To this end, the injection control module 115 may perform following functions.

The injection control module 115 may generate a drug injection condition based on input information on the drug injector 100 or user input information input by the user terminal 200, set a pulse sequence in which at least one or more pulse blocks having at least one or more preset voltage or current levels are sequentially arranged and which is applied to the driver of the drug injector 100 or the drug injection pump, based on a target drug injection amount included in the drug injection condition, and set the pulse sequence such that the total sum of the drug injection amounts to be supplied by each pulse block included in the pulse sequence corresponds to the target drug injection amount.

In this case, the input information may include at least one of a user's biometric information used for drug injection, a target drug injection amount, a target blood sugar level, a target drug injection period, and a target drug injection speed. In addition, the input information may include at least one of the target drug injection amount, the drug injection speed, or the user's biometric information input by the user through the user terminal connected to the drug injector. In addition, the injection control module 115 may derive the target drug injection amount based on the input information.

The injection control module 115 may read out and set a pulse sequence previously stored for each target drug injection amount from the memory. For example, an optimal pulse sequence may be designed in advance for each target drug injection amount and be stored in the memory in the form of a lookup table, and then, when the target drug injection amount is reached, a corresponding pulse sequence may be read out to be used from the lookup table.

In addition, the injection control module 115 may select a pulse block that supplies the maximum drug injection amount that is less than or equal to the target drug injection amount among the plurality of pulse blocks as a priority pulse block, and may set a pulse sequence such that the priority pulse block is arranged at least once but does not exceed the target drug injection amount.

In addition, when the total amount of drug injection amounts to be supplied by the priority pulse blocks is less than the target drug injection amount, the injection control module 115 may select a pulse block, which supplies the maximum drug injection amount that is less than or equal to the remaining drug injection amount, as a second-best pulse block, and may dispose the selected pulse block at least once.

The injection control module 115 may set a pulse sequence such that a certain time interval exists between the pulse blocks.

In this case, when the driver 121 uses an electroosmotic pump, each of the pulse blocks may be defined by a voltage value and voltage application time applied to the electroosmotic pump. The pulse block may include a stabilization time for which stabilization of the electroosmotic pump is performed. The pulse block may be defined by a forward voltage, a reverse voltage, and voltage application time applied to the electroosmotic pump, and the stabilization time may refer to the time for which stabilization of the electroosmotic pump is performed by applying 0 V between forward voltage application time and reverse voltage application time.

In addition, the pulse block may be defined by a forward current, a reverse current, and current application time applied to the electroosmotic pump, and the stabilization time may refer to the time for which stabilization of the electroosmotic pump is performed by applying 0 A between forward current application time and reverse current application time.

The safety control module 116 determines whether the drug injector 100 is in a state of being able to stably inject drug into the body by using multiple signals measured by the sensing unit 130 to monitor an operation state of the drug injector 100. Therefore, the safety control module 116 may stop drug injection or generate a notification to a user when it is determined that a drug injection operation of the drug injector 100 is not performed stably. Here, the notification may be generated by using a method by which the drug injector 100 may draw a user's attention by using sound, vibration, low-frequency stimulation, or so on.

In addition, the safety control module 116 may display to a user the type of notification, such as an alarm notification to notify the user to immediately stop drug injection of the drug injector 100 when a drug injection operation of the drug injector 100 is not performed normally, a caution notification to notify the user in advance when the drug injector 100 approaches a drug injection stop condition, and a notification display to simply displays a current state of the drug injector 100.

FIG. 4 is a conceptual diagram illustrating a function of the processor 240 according to an embodiment of the present disclosure. Hereinafter, an operation of the processor 240 according to an embodiment of the present disclosure is described with reference to FIG. 4.

The processor 240 may include an injection condition setting module 241, a drug injector management module 243, and a data management module 244, and may further include a pulse sequence setting module 242. The injection condition setting module 241 performs a function of setting a drug injection amount, a drug injection speed, a drug injection period, and so on of the drug injector 100. Specifically, the injection condition setting module 241 may generate an injection information input interface such that a user may input information on setting the drug injection amount, or may derive the drug injection amount by using at least one of the user's input information and the user's biometric information. In addition, the injection condition setting module 241 may generate a notification generation signal or a drug injection stop signal by monitoring a state of the drug injector 100.

The injection condition setting module 241 performs a function of receiving or managing information on drug injection, such as an injection amount threshold for each user used for an injection program or injection calculator, an injection amount threshold to be used for the drug injector 100, and user information. In addition, the injection condition setting module 241 may perform a function of automatically calculating a user's blood sugar level, an injection amount, a drug injection period, and so on to be displayed to a user.

The injection condition setting module 241 may generate a base injection calculator interface, a temporary base injection calculator interface, and a bolus injection calculator interface to be provided to a user. The base injection calculator interface may include an information input field for setting a time interval and speed such that a user may set the time interval or input a drug injection speed for each time interval. The base injection calculator interface may provide a user with a recommended drug injection speed for each time interval by using the user's input information, biometric data, drug injection history data, and so on.

The temporary base injection calculator interface may include information on temporarily changing a drug injection speed of base injection, such as whether a user exercises or decreases in activity level. Therefore, the temporary base injection calculator interface may include an information input field related to a user's exercise time, type of exercise, exercise intensity, decrease in activity level, and so on. The temporary base injection calculator interface may provide a period of time and a recommended injection speed required for the temporary base injection by using a user's input information, biometric data, exercise-related information, drug injection history data, and so on.

The bolus injection calculator interface may include a correction bolus calculation interface and a meal bolus calculation interface. The correction bolus calculator interface may include a blood sugar input field such that a user may input blood sugar information. The correction bolus calculator interface may derive a correction bolus injection amount for correcting a user's blood sugar level to a target blood sugar level when the blood sugar level input by the user is above a threshold value and provide the user with the correction bolus injection amount. The meal bolus calculator interface may include a meal information input field in which a user may input meal time, food intake information, carbohydrate intake amount, blood sugar information, and so on. The meal bolus calculator interface may provide a user with a recommended drug injection amount, a recommended drug injection speed, and a recommended drug injection period to maintain the user's blood sugar level within the target blood sugar range by using the user's meal information.

In addition, the injection condition setting module 241 may perform a function of transmitting a drug injection signal to the drug injector 100 by using the derived drug injection amount, drug injection speed, drug injection period, and so on.

The drug injector management module 243 may receive data related to an operation of the drug injector from the sensing unit 130 of the drug injector 100 and determine whether the drug injector 100 operates normally by using the received data. For example, the drug injector management module 243 may determine whether the drug injector 100 operates normally by comparing one or more pieces of the temperature data, pressure data, battery data, and drug remaining amount data of the drug injector 100 with a preset threshold. When one or more of the temperature data, pressure data, battery data, and drug remaining amount data of the drug injector 100 does not meet the preset threshold, the drug injector management module 243 may generate an alarm notification to provide to a user or stop the drug injection operation of the drug injector 100.

The drug injector management module 243 may generate an operation caution or operation alarm notification of the drug injector 100 by determining whether the drug injector 100 operates normally, or may stop an operation of the drug injector 100 and generate a drug injection stop notification. In addition, the drug injector management module 243 may perform a function of displaying the injection mode in addition to displaying operation states (normal, stop, and abnormal) of the drug injector 100.

The drug injector management module 243 is configured to execute the drug injector monitoring program to perform following functions and procedures. Accordingly, the drug injector management module 243 may monitor whether the drug injector satisfies a safe operation condition for stable operation, and generate an alarm notification when the drug injector deviates from the safe operation condition, or cause the drug injection operation to be stopped when the drug injector 100 does not meet a limit operation condition.

The drug injector management module 243 may obtain at least one piece of state information on the operation of the drug injector 100 from the drug injector 100. In addition, the drug injector management module 243 may output a notification signal on a state of the drug injector or output a control signal to stop an operation of the drug injector based on a result of comparing the state information with a reference value for each state information.

In this case, the state information may include at least one piece of the information on a battery remaining amount, a drug remaining amount, temperature of a specific region in the drug injector 100, or pressure of a specific region in the drug injector 100 measured by the sensing unit 130 of the drug injector 100.

The drug injector management module 243 may output a notification signal when an hourly change rate of at least one of the battery remaining amount, the drug remaining amount, temperature data, and pressure data is greater than or equal to a reference change rate.

The drug injector management module 243 may output a control signal to stop drug injection of the drug injector 100 when the temperature of the drug injector 100 is greater than or equal to a preset maximum temperature value or less than or equal to a preset minimum temperature value.

The drug injector management module 243 may output an alarm notification signal when a battery level of the drug injector 100 is lower than or equal to a first battery level, and may output a control signal to stop drug injection of the drug injector 100 when the battery level of the drug injector 100 is lower than or equal to a second battery level that is lower than the first battery level.

The drug injector management module 243 may output an alarm notification signal when a drug level of the drug injector 100 is lower than or equal to a first drug level, and may output a control signal to stop drug injection of the drug injector 100 when the drug level of the drug injector 100 is lower than or equal to a second drug level that is lower than the first drug level.

The drug injector management module 243 may output a control signal to perform a preset flow path blockage preventing operation when the pressure of the drug injector 100 is higher than a first pressure value, and may output an alarm notification signal and a control signal to stop drug injection of the drug injector when the pressure of the drug injector 100 is higher than a second pressure value that is higher than the first pressure value.

Here, the flow path blockage preventing operation may mean performing a drug injection operation by increasing a drug injection amount or a volume of the injected drug of the drug injector 100. In addition, the flow path blockage operation may include temporarily increasing a drug injection speed of the drug injector 100. For example, the flow path blockage preventing operation may mean that, when the drug injector 100 performs a drug injection operation by using an electric pulse block corresponding to the first drug injection amount, the drug injection operation is performed by using an electric pulse block corresponding to the second drug injection amount having a drug injection amount greater than or equal to the first drug injection amount, or an injection drug volume.

As the drug injection amount and the volume of the injected drug increase, the drug injection operation is performed at a greater pressure, and because the amount and volume of the drug moving through the drug injector 100 increase, foreign substances in a flow path may be removed, or the flow path may be prevented from being blocked.

The drug injector management module 243 may simultaneously receive a battery level and a drug level of the drug injector 100 and temperature and pressure data of a specific region in the drug injector 100 from the drug injector 100.

The drug injector management module 243 may receive the battery level and drug level of the drug injector 100 and the temperature and pressure data of a specific region in the drug injector 100 from the drug injector 100 only during a period when the drug injector 100 performs a drug injection operation.

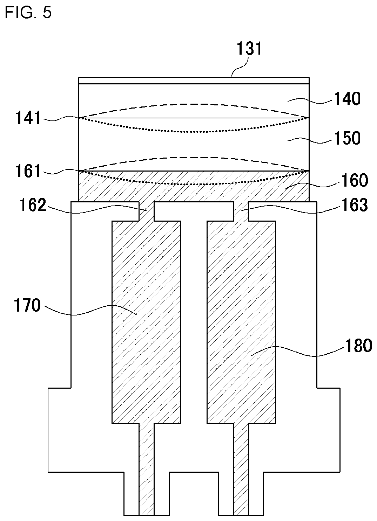

for example, the drug injector 100 may include an electroosmotic pump 150 (see FIG. 5), and the temperature of the specific region may be the temperature of the electroosmotic pump 150. In the same example, the pressure of the specific region may be the pressure of the electroosmotic pump 150.

The data management module 244 performs a function of storing and managing data related to drug injection. The data management module 244 may store data related to an operation of a patch or related to drug injection, or store user information. For example, the data management module 244 may store all data generated by an operation of the drug injector 100, such as blood sugar level input information, drug injection information, carbohydrate input information, and notification generation information. The data management module 244 may be linked to the data storage 300 to accumulate data for a certain period of time and transmit the accumulated data to the data storage 300.

In addition, the data management module 244 may not only simply store data, but also encrypt user personal information and so on. In addition, the data management module 244 may manage user accounts (IDs), passwords, user personal information, and so on for the operation of the drug injector 100.

The pulse sequence setting module 242 may set a pulse sequence for controlling the driver 121 of the drug injector 100 by using the drug injection condition derived by the injection condition setting module 241. That is, the pulse sequence setting module 242 may perform the same function as the injection control module 115 of the drug injector 100. Therefore, the pulse sequence may be set by using either the processor 113 or 240 included in the drug injector 100 or the user terminal 200.

A structure of the drug injector 100 is described below with reference to FIG. 5.

The drug injector 100 may include a pressure measurer including a pressure chamber 140 and a sensor 131, the electroosmotic pump 150, a transfer chamber 160, a first isolator 141, a second isolator 161, a suction path 162, a discharge path 163, a suction passage 170, and a discharge passage 180.

The electroosmotic pump 150 includes a membrane, a first electrode arranged on one side of the membrane, and a second electrode arranged on the other side of the membrane. As polarities of a voltage or current are alternately supplied to the first electrode and the second electrode, forward and reverse electrochemical reactions occur repeatedly. Accordingly, a fluid inside the electroosmotic pump 150 moves back and forth, thereby generating pressure for transferring drug.

The first isolator 141 and the second isolator 161 that seal the electroosmotic pump 150 may be provided respectively on both sides of the electroosmotic pump 150. The first isolator 141 and the second isolator 161 change in shape according to the positive pressure or negative pressure generated by the electroosmotic pump 150. The change in shape of the first isolator 141 causes a change in pressure in the pressure chamber 140. The change in shape of the second isolator 161 causes a change in pressure in the transfer chamber 160. That is, the pressure generated by the electroosmotic pump 150 is transferred to the pressure chamber 140 and the transfer chamber 160 by using the first isolator 141 and the second isolator 161.

A passage through which drug moves may be formed on one side of the transfer chamber 160. Specifically, the suction passage 170 may include a drug storage or may be connected to the drug storage. When negative pressure is generated by the electroosmotic pump 150, drug moves to the transfer chamber 160 through the suction passage 170 and the suction path 162. In this case, the second isolator 161 may prevent the drug from flowing into the electroosmotic pump 150.

In addition, the discharge passage 180 may include an injection device for injecting the drug into the body or may be connected to the injection device. When positive pressure is generated by the electroosmotic pump 150, the drug stored in the transfer chamber 160 moves through the discharge path 163 and the discharge passage 180.

The pressure measurer 140 and 131 may be disposed on the other side of the electroosmotic pump 150. That is, the pressure measurer 140 and 131 is disposed on an opposite side of a path through which drug moves or disposed on a surface that is not in contact with the path through which the drug moves, based on the electroosmotic pump 150.

The pressure measurer 140 and 131 may include the pressure chamber 140 and the pressure sensor 131. The pressure chamber 140 refers to a space through which the pressure generated by the electroosmotic pump 150 is transferred. The pressure sensor 131 may measure the pressure of the pressure chamber 140 and detect blockages of the suction path 162, the discharge path 163, the suction passage 170, and the discharge passage 180.

Specifically, the electroosmotic pump 150 generates pressure alternately. Accordingly, the pressure transferred to the transfer chamber 160 and the pressure transferred to the pressure chamber 140 have a constant correlation. When the suction path 162, the discharge path 163, the suction passage 170, and the discharge passage 180 through which drugs move are blocked or abnormal, the pressure changes. Accordingly, a change in pressure transferred to the pressure chamber 140 occurs. The pressure measurer 140 and 131 measures the change in pressure and detects blockage and abnormality in flow paths.

Data measured by the drug remaining amount measurement module 134, the pressure sensor 131, the temperature sensor 132, and the battery sensor 133 may be transmitted to the user terminal 200 by a communication module. The user terminal 200 determines whether the drug injector 100 operates under a safe operation condition in which a stable operation may be performed, based on the received data. When the drug injector 100 deviates from the safe operation condition, an alarm notification is generated and transferred to a user or the drug injection operation of the drug injector 100 is stopped to prevent excessive or insufficient drug from injecting to the user.

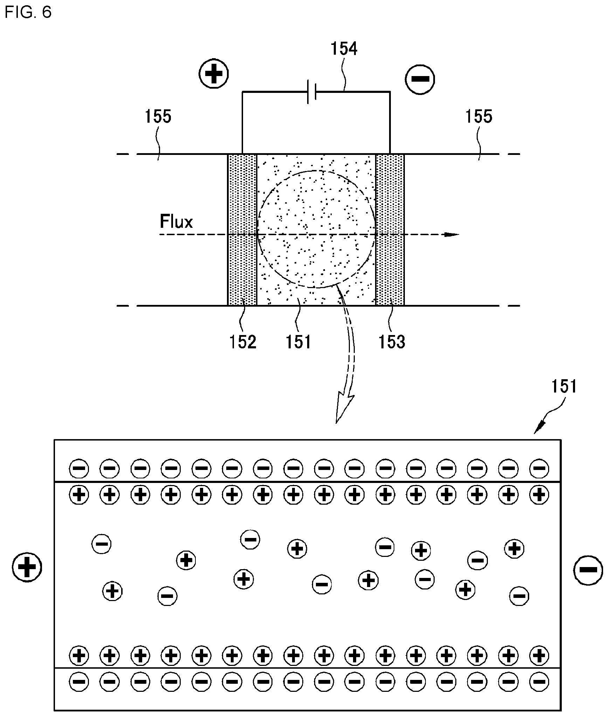

Hereinafter, a configuration of the electroosmotic pump 150 according to the embodiment of the present disclosure is described in more detail with reference to FIG. 6.

Referring to FIG. 6, the drug injector 100 according to the embodiment of the present disclosure may include the electroosmotic pump 150 that generates pressure for transferring a drug. The electroosmotic pump 150 uses movement of a fluid made by an electroosmotic phenomenon that occurs when a voltage or current is applied on both ends of a porous membrane 151 through electrodes. The electroosmotic pump 150 may include the membrane 151, a positive electrode 152 and a negative electrode 153 arranged on both sides of the membrane 151, a power source 154 that applies a voltage or current to the electrodes, and a flow path 155 for fluid movement.

Silica, glass, and so on are generally used as a material of the porous film 111a, and when the silica and glass are immersed in an aqueous solution, surfaces thereof are negatively charged. In this state, when a voltage or current is applied, a fluid moves from the positive electrode 152 to the negative electrode 153. There are many paths, through which fluids may pass, in the porous film 151, and when one of the paths is enlarged, a surface of the fluid path with negative charges (bound anions) may be in a pseudostate in which the negative charges are balanced by mobile cations having positive charges.

When a voltage or current is applied under the condition, mobile cations move along the surface from the positive electrode 152 to the negative electrode 153, and accordingly, a phenomenon occurs in which the entire fluid connected by a hydrogen bond network flows as if slipping, and this phenomenon is called electroosmosis, and a pump that uses the phenomenon is an electroosmotic pump.

In order to facilitate movement of a fluid, various electrode materials, such as a platinum mesh that is a porous electrode, porous carbon paper or carbon cloth, or a porous structure coated on top of the electrode, as well as various fixable materials applied to an impermeable base material through drop coating or spin coating, may be used as the electrodes used for the electroosmotic pump.

The electroosmotic pump 150 supplies a voltage or current, by alternating polarities thereof, to the positive electrode 152 and the negative electrode 153, and thereby, forward and reverse electrochemical reactions occur reversibly. As the forward and reverse electrochemical reactions occur repeatedly, the second fluid inside the electroosmotic pump repeatedly performs a reciprocating motion. In addition, by the repetitive forward and reverse reversible electrochemical reactions, the positive electrode 152 and the negative electrode 153 are repeatedly consumed and regenerated.

Therefore, when the drug injector 100 includes the electroosmotic pump 150, the drug injector monitoring system may control the pressure generated by the electroosmotic pump 150 and a pulse volume of the discharged drug by controlling a magnitude and application of the voltage or current applied to the positive electrode 152 and the negative electrode 153.

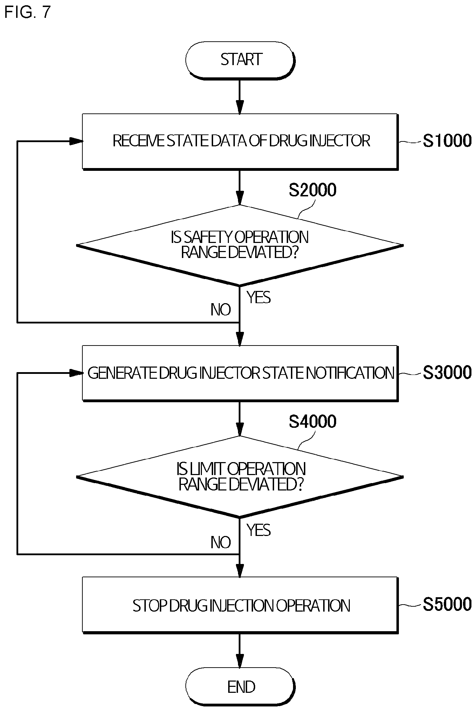

Hereinafter, a drug injector monitoring method according to an embodiment of the present disclosure is described with reference to FIGS. 7 to 11. Steps described below may be performed by the user terminal 200 and the processor 240 of the user terminal 200, or the drug injector 100 and the processor 113 of the drug injector 100. Therefore, the embodiments of the present disclosure described above with reference to FIGS. 1 to 6 may also be applied to the embodiments described below.

Referring to FIG. 7, the drug injector monitoring method according to the present disclosure includes step S1000 of receiving data of a drug injector, step S2000 of determining whether to deviate a safe operation range, and step S3000 of a generating drug injector state notification.

The drug injector monitoring method according to the present disclosure compares a battery remaining amount, a drug remaining amount, and temperature and pressure data of a specific region in the drug injector 100 with respective reference values, and provides a notification on a state of the drug injector 100 or stops a drug injection operation of the drug injector 100 based on a result of comparison. Here, the reference values include a safe operation condition which is a reference value for providing a notification to a user, and a limit operation condition which is a reference value for stopping a drug injection operation of the drug injector 100.

In step S1000 of receiving data of the drug injector 100, at least one piece of state information on an operation of the drug injector 100 is acquired by the drug injector 100. The state information may include at least one of battery remaining data and drug remaining data of the drug injector 100, temperature data of a specific region in the drug injector 100, and pressure information of a specific region in the drug injector 100.

In step S1000 of receiving the data of the drug injector 100, in order to reduce battery consumption of the drug injector 100, data measurement and reception times may be synchronized or data measurement and reception may be performed according to a preset order.

For example, transmission and reception times of the battery remaining data, the drug remaining data, the temperature data, and the pressure data of the drug injector 100 may be synchronized to receive the data simultaneously, or may be transmitted and received according to a preset order, and thereby, power consumption for data transmission may be reduced.

Alternatively, power consumption may be reduced by measuring the battery remaining data, the drug remaining data, the temperature data, and the pressure data only during a period in which the drug injector 100 operates or performs a drug injection operation.

In step S2000 of determining whether to deviate a safe operation condition, the state information is compared with a reference value for each state information. For example, the battery remaining data, the drug remaining data, the temperature data, and the pressure data are respectively compared with the preset safe operation conditions. The safe operation conditions refer to operation conditions for the drug injector 100 to stably inject an accurate amount of drug. Therefore, in addition to the battery remaining amount, the drug remaining amount, temperature, and pressure, various types of data for the drug injector 100 to stably inject an accurate amount of drug may be included.

In step S3000 of generating a state notification of the drug injector 100, when at least one of the battery remaining amount data, the drug remaining amount data, the temperature data, and the pressure data deviates from the safe operation conditions, a notification is provided to a user by using the user terminal 200 or the drug injector 100. The notification may include not only a notification using sound and display, but also a notification using vibration, a low-frequency stimulation signal, and display. In addition, the notification may be provided to a user by classifying types of the notifications by changing the number or intensity of notification signals ,such as sound, vibration, a low-frequency stimulation signal, and display brightness.

In addition, step S2000 of determining whether to deviate the safe operation condition and step S3000 of generating the state notification of the drug injector 100 may include a step of generating a notification when an hourly change amount of one or more of the operation conditions is greater than or equal to a reference change rate. That is, step S2000 of determining whether to deviate the safety operation condition may further include a step of determining whether at least one of the battery remaining amount data, the drug remaining amount data, the temperature data, and the pressure data has an hourly change rate greater than or equal to the reference change rate. The reference change rates for the battery remaining amount, the drug remaining amount, temperature, and pressure may each be individually set.

Step S3000 of generating the state notification of the drug injector 100 may further include a step of generating a notification when at least one of the battery remaining amount data, the drug remaining amount data, the temperature data, and the pressure data has an hourly change rate greater than or equal to the reference change rate. Accordingly, a user may recognize whether the safe operation conditions of the drug injector 100 rapidly change and may check or prevent abnormal operation and failure of the drug injector 100.

When the hourly change rate of any one or more of the battery remaining amount, the drug remaining amount, temperature, and pressure is greater than or equal to the reference change rate, there is a possibility that abnormality occurs in the conditions for the drug injector 100 to perform a drug injection operation stably or a failure occurs in a device. Therefore, by notifying a user that abnormality occurs in the safe operation conditions of the drug injector 100, malfunction of the drug injector 100 may be detected and prevented.

The drug injector monitoring method according to the present disclosure may further include step S4000 of determining whether to deviate a limit operation range and step S5000 of stopping a drug injection operation.

In step S4000 of determining whether to deviate the limit operation condition, battery remaining amount data, drug remaining amount data, temperature data, and pressure data are respectively compared with preset limit operation conditions. The limit operation conditions refer to operation conditions that may have a negative effect on a user or inject drug exceeding a preset error range when the drug injector 100 performs the drug injection operation. Therefore, in addition to the battery remaining amount, the drug remaining amount, the temperature, and the pressure, various types for the drug injector 100 to stably inject an accurate amount of drug may be included.

In step S5000 of stopping the drug injection operation, when at least one of the battery remaining amount data, the drug remaining amount data, the temperature data, and the pressure data exceeds the limit operation condition, the drug injector 100 is controlled to stop the drug injection operation. When the drug injection operation stops, a drug injection operation stop notification may be provided to a user by the user terminal (200) or the drug injector 100.

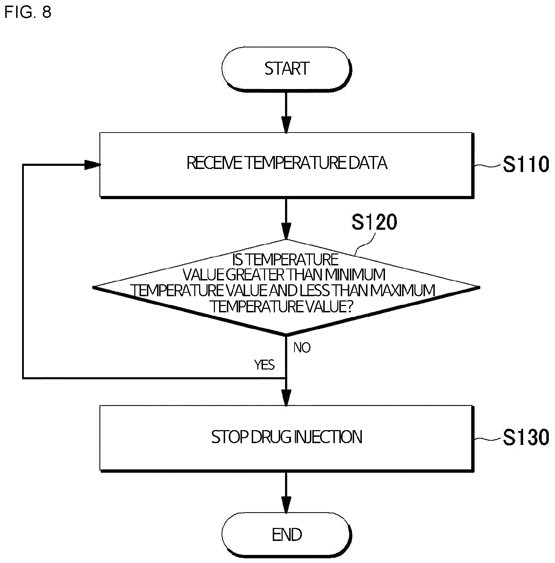

Hereinafter, a method for stopping the drug injection operation and generating a notification according to temperature data is described with reference to FIG. 8.

Step S110 of receiving temperature data refers to a step of receiving temperature data of the drug injector 100. Therefore, step S110 of receiving the temperature data may be included in step S1000 of receiving data of the drug injector 100.

In step S120 of comparing operation conditions, it is determined whether a temperature value corresponding to the received temperature data is within a preset operation temperature range. The minimum temperature may be set to any value within a range of 10 to 20 degrees and the maximum temperature of 40 to 50 degrees but is not limited thereto and may be appropriately changed depending on types of drug, specifications of an electroosmotic pump, an electrode reaction speed of the electroosmotic pump, and so on.

For example, when the temperature of the drug injector 100 increases, a difference in gas solubility of drug stored in the drug injector 100 may occur to increase the possibility of bubble generation. When the possibility of bubble generation increases, the probability of error occurring between the set drug injection amount and the actual drug injection amount increases. In addition, when the temperature is too high or too low, the performance of a battery may be affected. Therefore, a minimum temperature value and a maximum temperature value of the drug injector 100 may be set based on the gas solubility of drug according to the temperature.

In step S130 of stopping the drug injection, when a temperature value of the drug injector 100 is out of the minimum temperature value and maximum temperature value range, the drug injector 100 is controlled to stop a drug injection operation. That is, the power applied to an electroosmotic pump of the drug injector 100 may be disconnected.

When the drug injection is stopped, an alarm notification is generated such that a user may detect danger. In addition to the method of displaying the alarm notification by using the user terminal 200, the alarm notification may include a method of controlling the drug injector 100 such that the alarm notification is generated.

In addition, the alarm notification is not limited to a method of displaying the contents of the alarm notification to a user through a display, and may include a method of generating various stimulus signals, such as sound, vibration, and low-frequency stimulus signals to draw a user's attention. The alarm notification may also be applied to alarm notifications based on battery remaining amount, drug remaining amount, and pressure described below.

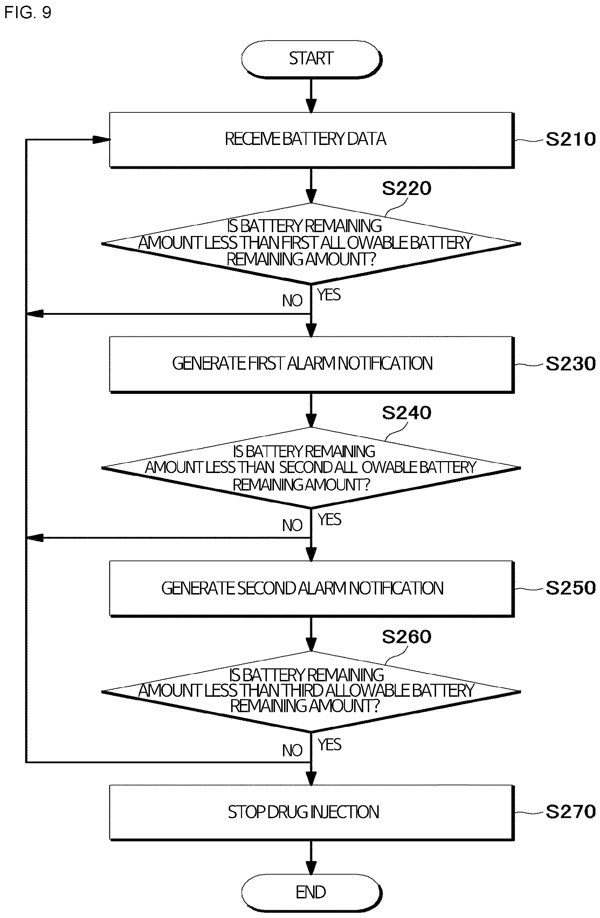

Hereinafter, a method of stopping drug injection operation and generating an alarm notification according to battery remaining amount data will be described with reference to FIG. 9.

Step S210 of receiving battery data refers to a step of receiving battery data of the drug injector 100. Therefore, step S210 of receiving the battery data may be included in step S1000 of receiving the data of the drug injector 100.

In step S220 of comparing first operation conditions, it is determined whether a battery remaining value corresponding to the received battery data is less than a preset first allowable battery remaining value. The first allowable battery remaining value corresponds to a value randomly set by a user. Therefore, the first allowable battery remaining value may refer to a battery remaining value that a user wants to receive a notification.

In step S230 of generating a first alarm notification, when a battery remaining value of the drug injector 100 is less than the first allowable battery remaining value, the first alarm notification is generated. A user notification may refer to a notification indicating that the battery remaining value of the drug injector 100 reaches the first allowable battery remaining value set by a user.

In step S240 of comparing a second operation condition, it is determined whether a battery remaining value corresponding to the received battery data is less than a preset second allowable battery remaining value. The second allowable battery remaining value refers to a battery remaining value for notifying a user of the risk of drug injection stop when the battery remaining value approaches a value at which a drug injection operation of the drug injector 100 has to be stopped.

The second allowable battery remaining value may refer to the degree at which the drug injector 100 may be at risk of not being able to operate stably when the battery is rapidly consumed due to changes, such as temperature, the increased number of operations, and the increased number of communications.

The second allowable battery remaining value may be set to about 30 % of the total battery capacity but is not limited thereto and may be set to any one of the values greater than or equal to a third allowable battery remaining value.

In step S250 of generating a second alarm notification, when a battery remaining value of the drug injector 100 is less than the second allowable battery remaining value, an alarm notification is generated such that a user may detect the risk. The first alarm notification and the second alarm notification are expressed differently from each other, and accordingly, a user may distinguish between the first alarm notification and the second alarm notification. That is, one or more of a melody, a frequency, and a volume of the notification, the number of notifications, a vibration frequency, vibration intensity, intensity of a low-frequency electrical stimulation signal, and a frequency of the low-frequency electrical stimulation signal are set differently.

In step S260 of comparing a third operation condition, it is determined whether a battery remaining value corresponding to the received battery data is less than a preset third allowable battery remaining value. The third allowable battery remaining value may refer to a value less than or equal to the second allowable battery remaining value. The third allowable battery remaining value may refer to a battery remaining value at which the drug injector 100 is likely to stop operating while performing a drug injection operation. In addition, the third allowable battery remaining value may refer to a battery remaining value at which the drug injector 100 is likely to stop operating while performing a drug injection operation or a battery remaining value that may have a negative effect on the drug injector 100 when the drug injector 100 operates in the current state. The third allowable battery remaining value may be set to about 10 % of the total battery capacity but is not limited thereto and may be set to any one of the values less than or equal to the second allowable battery remaining value.

In step S270 of stopping the drug injection, when a battery remaining value of the drug injector 100 exceeds the third allowable battery remaining value, the drug injector 100 stops a drug injection operation. Accordingly, it is possible to prevent a situation in which the drug injection operation of the drug injector 100 is stopped due to the power being exhausted during the drug injection operation, causing an error in the drug injection amount.

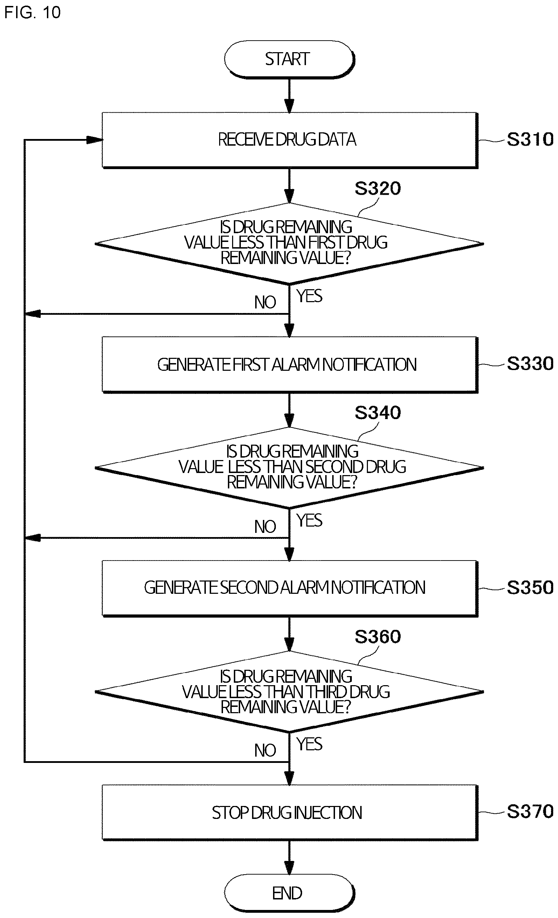

Hereinafter, a method of stopping a drug injection operation and generating an alarm notification according to the drug remaining amount data will be described with reference to FIG. 10.

Step S310 of receiving drug data refers to a step of receiving drug data of the drug injector 100. Therefore, step S310 of receiving the drug data may be included in step S1000 of receiving the data of the drug injector 100.

In step S320 of comparing a first operation condition, it is determined whether a drug remaining amount corresponding to the received drug data is less than a first drug remaining amount set previously. The first drug remaining amount corresponds to a value randomly set by a user. Therefore, the first drug remaining amount may refer to a drug remaining amount for which a user wants to receive a notification. The first drug remaining amount may be set to 20 U (200 *µ*ℓ) for insulin but is not limited thereto and may be changed depending on specifications of the electroosmotic pump, the type of drug or insulin, an injection control method, and so on.

In step S330 of generating a first alarm notification, when a drug remaining amount of the drug injector 100 is less than a first drug remaining amount, the first alarm notification is generated such that a user may detect the risk.

In step S340 of comparing a second operation condition, it is determined whether a drug remaining amount corresponding to the received drug data is less than a preset second drug remaining amount. The second drug remaining amount refers to the degree to which the drug injector 100 may not inject a sufficient amount of drug when drug is rapidly consumed due to changes, such as temperature and an increase in the number of injections. In addition, the second drug remaining amount may refer to a drug remaining amount that requires a user's attention as the drug remaining amount decreases.

The second drug remaining amount may be set to 13 U (130 *µ*ℓ) for insulin but is not limited thereto and may be set to any value higher than or equal to a third drug remaining amount.

In step S350 of generating a second alarm notification, when a drug remaining amount of the drug injector 100 is less than the second drug remaining amount, the second alarm notification is generated such that a user may detect the risk. As described above, the first alarm notification and the second alarm notification are expressed differently, and accordingly, a user may distinguish between the first alarm notification and the second alarm notification.

In step S360 of comparing a third operation condition, it is determined whether a drug remaining value corresponding to the received drug data is less than a preset third drug remaining value. The third drug remaining value refers to a drug remaining value at a level where the drug injector 100 may not inject a sufficient amount of drug.

In addition, the third drug remaining value may refer to a value less than or equal to the second drug remaining value. The third drug remaining value may be set to about 8 U (80 *µ*ℓ), when drug is insulin, but is not limited thereto, and may be set to any value less than or equal to the second drug remaining value.

In step of stopping drug injection, when a drug remaining value of the drug injector 100 exceeds a third drug remaining value, the drug injector 100 stops a drug injection operation. Therefore, it is possible to prevent a situation in which the drug injector 100 fails to inject a sufficient amount of drug due to the drug remaining amount being depleted during the drug injection operation, thereby causing an error in the drug injection amount.

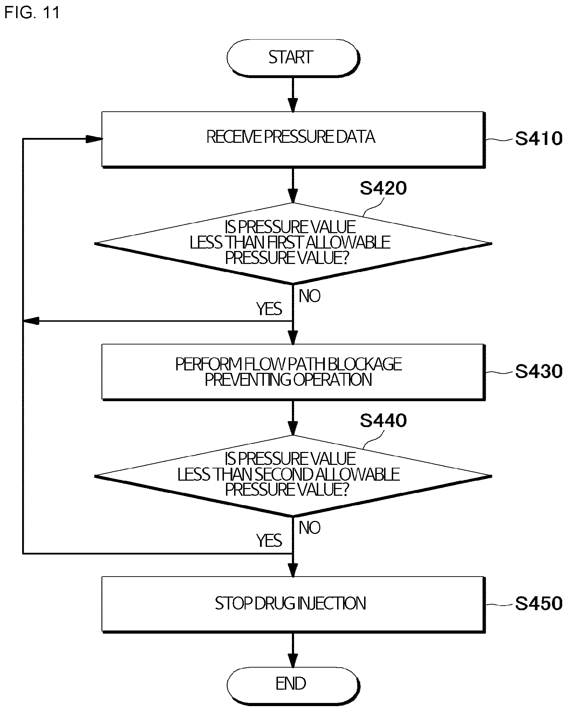

Hereinafter, a method of stopping a drug injection operation and generating an alarm notification according to pressure data will be described with reference to FIG. 11.

Step S410 of receiving pressure data refers to a step of receiving pressure data of the drug injector 100. Therefore, step S410 of receiving the pressure data may be included in step S1000 of receiving the data of the drug injector 100.

In step S420 of comparing a first operation condition, it is determined whether a pressure value corresponding to the received pressure data is less than a preset first allowable pressure value. The first allowable pressure value may be set to a value of 10.0 kPa or more but is not limited thereto and may be changed depending on specifications of an electroosmotic pump, the type of drug, temperature, and a volume of the drug to be injected.

For example, when the volume of the drug to be input is 0.5 *µ*ℓ, the first allowable pressure value may be set to a value of 10.0 kPa or higher, and when the volume of the drug to be input is 1.0 *µ*ℓ, the first allowable pressure value may be set to a value of 16.0 kPa or higher, and when the volume of the drug to be input is 3.0 *µ*ℓ, the first allowable pressure value may be set to a value of 70.0 kPa or higher.

In step S430 of performing a flow path blockage preventing operation, when a pressure value of the drug injector 100 is less than the first allowable pressure value, step S410 of receiving the pressure data is repeated. When the pressure value of the drug injector 100 is greater than or equal to the first allowable pressure value, it is determined that there is a risk of flow path blockage, and a flow path blockage preventing operation is performed. The flow path blockage preventing operation refers to an operation of removing or preventing blockage of a flow path through which drug moves, such as increasing an injection speed of the drug to be injected or increasing an injection amount of the drug.

In step S430 of performing the flow path blockage preventing operation, when the pressure value of the drug injector 100 is higher than or equal to the first allowable pressure value, an alarm notification may be generated such that a user may detect the risk.

In step S440 of comparing a second operation condition, it is determined whether a pressure value corresponding to the received pressure data is less than a preset second allowable pressure value. The second allowable pressure value may refer to a pressure value higher than or equal to the first allowable pressure value. The second allowable pressure value refers to a pressure value that may cause an error in the drug injection amount due to blockage of a flow path, which may have a negative effect on a user's health when the drug injector 100 performs drug injection outside the second allowable pressure value.

For example, when the volume of the drug to be input is 0.5 *µ*ℓ, the second allowable pressure value may be set to a value of 12.0 kPa or higher, and when the volume of the drug to be input is 1.0 *µ*ℓ, the second allowable pressure value may be set to a value of 19.5 kPa or higher, and when the volume of the drug to be input is 3.0 *µ*ℓ, the second allowable pressure value may be set to a value of 83.0 kPa or higher.

In step S450 of stopping drug injection, when a pressure value of the drug injector 100 is less than the second allowable pressure value, step S410 of receiving the pressure data is repeated. When the pressure value of the drug injector 100 is greater than or equal to the second allowable pressure value, the drug injector 100 stops the drug injection operation. Therefore, it is possible to prevent a situation in which drug is injected while there is an error between a set drug injection amount and an actually injected drug amount due to blockage of a flow path.

One embodiment of the present disclosure may also be implemented in the form of a recording medium including computer-executable instructions, such as a program module executed by a computer. A computer readable medium may be any available medium that may be accessed by a computer and includes both volatile and nonvolatile media, removable and non-removable media. Also, the computer readable medium may include a computer storage medium. A computer storage medium includes both volatile and nonvolatile media and removable and non-removable media implemented by any method or technology for storing information, such as computer readable instructions, data structures, program modules or other data.

In addition, although the method and device of the present disclosure are described with respect to specific embodiments, some or all of components or operations thereof may be implemented by using a computer system having a general-purpose hardware architecture.

The above description of the present disclosure is for illustrative purposes only, and those skilled in the art will appreciate that the present disclosure may be easily modified into other specific forms without changing the technical idea or essential characteristics of the present disclosure. Therefore, the embodiments described above should be understood as illustrative in all respects and not limiting. For example, respective components described as a single entity may be implemented in a distributed manner, and likewise, components described as a distributed manner may be implemented in a combined manner.

The scope of the present disclosure is indicated by the claims described below rather than the detailed description above, and all changes or modified forms derived from the meaning, scope of the claims, and their equivalent concepts should be interpreted as being included in the scope of the present application.

## Claims

1. A drug injector monitoring method performed by a processor, the drug injector monitoring method comprising:
obtaining at least one or more pieces of state information on an operation of a drug injector from the drug injector; and
outputting one of a notification signal on a state of the drug injector and a control signal for stopping a drug injection operation of the drug injector based on a result of comparing the state information with a reference value for each piece of the state information,
wherein the state information includes a battery remaining amount of the drug injector, a drug remaining amount of the drug injector, temperature of a specific region in the drug injector, and or pressure of the specific region in the drug injector.

2. The drug injector monitoring method of claim 1, wherein
the outputting of one of the notification signal and the control signal includes outputting the notification signal when an hourly change rate of at least one of the battery remaining amount, the drug remaining amount, the temperature, and the pressure is greater than or equal to a reference change rate.

3. The drug injector monitoring method of claim 1, wherein
the outputting of one of the notification signal and the control signal includes outputting the control signal for stopping a drug injection of the drug injector when temperature of the drug injector is higher than or equal to a preset maximum temperature or is lower than or equal to a preset minimum temperature.

4. The drug injector monitoring method of claim 1, wherein
in the outputting of one of the notification signal and the control signal,
when the battery remaining amount of the drug injector is less than or equal to a first battery remaining amount, the notification signal is output, and
when the battery remaining amount of the drug injector is less than or equal to a second battery remaining amount that is less than the first battery remaining amount, the control signal for stopping the drug injection of the drug injector is output.

5. The drug injector monitoring method of claim 1, wherein, in the outputting of one of the notification signal and the control signal,
when the drug remaining amount of the drug injector is less than or equal to the first drug remaining amount value, the notification signal is output, and
when the drug remaining amount of the drug injector is less than or equal to the second drug remaining amount value that is less than the first drug remaining amount value, the control signal for stopping the drug injection of the drug injector is output.

6. The drug injector monitoring method of claim 1, wherein, in the outputting of one of the notification signal and the control signal,
when pressure of the drug injector is higher than a first pressure value, the control signal is output such that a preset flow path blockage preventing operation is performed, and
when the pressure of the drug injector is a second pressure value that is higher than the first pressure value, the notification signal and the control signal for stopping the drug injection operation of the drug injector are output.

7. The drug injector monitoring method of claim 1, wherein
in the obtaining of at least one or more pieces of state information, the battery remaining amount, the drug remaining amount, the temperature, and the pressure are simultaneously received from the drug injector.

8. The drug injector monitoring method of claim 1, wherein
in the obtaining of at least one or more pieces of state information, the battery remaining amount, the drug remaining amount, the temperature, and the pressure are received from the drug injector only during a period in which the drug injection operation of the drug injector is performed.

9. The drug injector monitoring method of claim 1, wherein
the drug injector includes an electroosmotic pump, and
the temperature of the specific region is temperature of the electroosmotic pump.

10. The drug injector monitoring method of claim 1, wherein
the drug injector includes an electroosmotic pump, and
the pressure of the specific region is the pressure of the electroosmotic pump.

11. The drug injector monitoring method of claim 1, wherein
the pressure of the specific region is pressure of a drug discharge portion of the drug injector.

12. A drug injector monitoring device comprising:
a memory storing a drug injector monitoring program; and
a processor configured to execute the drug injector monitoring program stored in the memory,
wherein the processor is further configured to execute the drug injector monitoring program to obtain at least one or more pieces of state information on an operation of a drug injector from the drug injector and to output one of a notification signal on a state of the drug injector and a control signal for stopping a drug injection operation of the drug injector based on a result of comparing the state information with a reference value for each piece of the state information, and
the state information includes a battery remaining amount of the drug injector, a drug remaining amount of the drug injector, temperature of a specific region in the drug injector, and or pressure of the specific region in the drug injector.

13. The drug injector monitoring device of claim 12, wherein
the processor outputs the notification signal when an hourly change rate of at least one of the battery remaining amount, the drug remaining amount, the temperature, and the pressure is greater than or equal to a reference change rate.

14. The drug injector monitoring device of claim 12, wherein
the processor outputs the control signal for stopping a drug injection of the drug injector when temperature of the drug injector is higher than or equal to a preset maximum temperature or is lower than or equal to a preset minimum temperature.

15. The drug injector monitoring device of claim 12, wherein
the processor outputs the notification signal when the battery remaining amount of the drug injector is less than or equal to a first battery remaining amount, and outputs the control signal for stopping the drug injection of the drug injector when the battery remaining amount of the drug injector is less than or equal to a second battery remaining amount that is less than the first battery remaining amount.

16. The drug injector monitoring device of claim 12, wherein
the processor outputs the notification signal when the drug remaining amount of the drug injector is less than or equal to the first drug remaining amount value, and outputs the control signal for stopping the drug injection of the drug injector when the drug remaining amount of the drug injector is less than or equal to the second drug remaining amount value that is less than the first drug remaining amount value.

17. The drug injector monitoring device of claim 12, wherein
the processor outputs the control signal such that a preset flow path blockage preventing operation is performed when pressure of the drug injector is higher than a first pressure value, and outputs the notification signal and the control signal for stopping the drug injection operation of the drug injector when the pressure of the drug injector is a second pressure value that is higher than the first pressure value.

18. The drug injector monitoring device of claim 12, wherein
the battery remaining amount, the drug remaining amount, the temperature, and the pressure are simultaneously received from the drug injector.

19. The drug injector monitoring device of claim 12, wherein
the battery remaining amount, the drug remaining amount, the temperature, and the pressure are received from the drug injector only during a period in which the drug injection operation of the drug injector is performed.

20. The drug injector monitoring device of claim 12, wherein
the drug injector includes an electroosmotic pump, and
the temperature of the specific region is temperature of the electroosmotic pump.

21. The drug injector monitoring device of claim 12, wherein
the drug injector includes an electroosmotic pump, and
the pressure of the specific region is the pressure of the electroosmotic pump.

22. The drug injector monitoring device of claim 12, wherein
the pressure of the specific region is pressure of a drug discharge portion of the drug injector.
